# EUROPEAN PATENT APPLICATION

(11) **EP 1 930 406 A1**
(43) Date of publication of application: **11.06.2008**
(21) Application number: 06783020.8
(22) Date of filing: 25.08.2006
(51) Int. Cl.: C12N 1/19, A61K 36/06, A61K 45/00, A61K 47/48, C07K 7/06, C12N 15/09

(54) **BRAIN-MIGRATING CELL AND USE THEREOF**

(30) Priority: 26.08.2005 JP 2005246256
(71) Applicant: Tissue Targeting Japan Inc., Nagoya-shi, Aichi 464-0858 (JP)
(72) Inventor: SAWADA, Makoto, Kasugai-shi, Aichi 487-0015 (JP); MUKAIYAMA, Hiroyuki, Nagoya-shi, Aichi 464-0846 (JP)
(74) Representative: Grünecker, Kinkeldey, Stockmair & Schwanhäusser Anwaltssozietät
(86) International application number: PCT/JP2006/316673
(87) International publication number: WO 2007/023930

(57) **Abstract**

It was discovered that cells expressing brain-localizing polypeptides on the cell surface can pass through the blood-brain barrier and therefore have the activity of translocating to the brain. By introducing desired substances into such brain-localizing cells, the substances can also be translocated to the brain. For example, by intravenously administering brain-localizing cells into which pharmaceutical agents having therapeutic effects on brain diseases have been introduced, such agents can be efficiently delivered into the brain and the brain diseases can be effectively treated.

SEQUENCE LISTING

<110>  TISSUE TARGETING JAPAN INC.

<120>  Brain-localizing cells and uses thereof

<130>  EP56948HV015pau

<140>  EP 06 783 020.8
<141>  2006-08-25

<150>  PCT/JP2006/316673
<151>  2006-08-25

<150>  JP 2005-246256
<151>  2005-08-26

<160>  14

<170>  PatentIn version 3.3

<210>  1
<211>  9
<212>  PRT
<213>  Artificial

<220>
<223>  an artificially synthesized sequence

<400>  1

Cys Ser Asn Leu Leu Ser Arg His Cys
1               5


<210>  2
<211>  9
<212>  PRT
<213>  Artificial

<220>
<223>  an artificially synthesized sequence

<400>  2

Cys Ser Leu Asn Thr Arg Ser Gln Cys
1               5


<210>  3
<211>  9
<212>  PRT
<213>  Artificial

<220>
<223>  an artificially synthesized sequence

<400>  3

Cys Val Ala Pro Ser Arg Ala Thr Cys
1               5


<210>  4
<211>  9
<212>  PRT
<213>  Artificial

<220>

<223>  an artificially synthesized sequence

<400>  4

Cys Val Val Arg His Leu Gln Gln Cys
1               5


<210>  5
<211>  9
<212>  PRT
<213>  Artificial

<220>
<223>  an artificially synthesized sequence

<400>  5

Cys Val Leu Arg His Leu Gln Gln Cys
1               5


<210>  6
<211>  9
<212>  PRT
<213>  Artificial

<220>
<223>  an artificially synthesized sequence

<400>  6

Cys Arg Gln Leu Val Gln Val His Cys
1               5


<210>  7
<211>  9
<212>  PRT
<213>  Artificial

<220>
<223>  an artificially synthesized sequence

<400>  7

Cys Gly Pro Leu Lys Thr Ser Ala Cys
1               5


<210>  8
<211>  9
<212>  PRT
<213>  Artificial

<220>
<223>  an artificially synthesized sequence

<400>  8

Cys Leu Lys Pro Gly Pro Lys His Cys
1               5


<210>  9
<211>  9
<212>  PRT
<213>  Artificial

<220>
<223>  an artificially synthesized sequence

<400>  9

Cys Arg Ser Pro Gln Pro Ala Val Cys
1               5


<210>  10
<211>  9
<212>  PRT
<213>  Artificial

<220>
<223>  an artificially synthesized sequence

<400>  10

Cys Asn Pro Leu Ser Pro Arg Ser Cys
1               5


<210>  11
<211>  9
<212>  PRT
<213>  Artificial

<220>
<223>  an artificially synthesized sequence

<400>  11

Cys Pro Ala Gly Ala Val Lys Ser Cys
1               5


<210>  12
<211>  9
<212>  PRT
<213>  Artificial

<220>
<223>  an artificially synthesized sequence

<400>  12

Cys Pro Ala Gly Ala Leu Lys Ser Cys
1               5


<210>  13
<211>  36
<212>  DNA
<213>  Artificial

<220>
<223>  an artificially synthesized primer sequence

<400>  13
ggtacctgcg tcttgagaca tctacaacaa tgttaa                                36


<210>  14
<211>  36
<212>  DNA
<213>  Artificial

<220>

<223>  an artificially synthesized primer sequence

<400>  14
ctcgagttaa cattgttgta gatgtctcaa gacgca                                36

## Description

### Technical Field

The present invention relates to cells conferred with brain-localizing activity and uses of such brain-localizing cells as carrier molecules for translocating desired substances to the brain.

### Background Art

Transport of substances and cells to the brain, the center of higher function, is restricted by a barrier structure called the blood-brain barrier. Therefore the brain was a site where it was difficult to conduct effective treatment, except by surgical operation. Even when white blood cells such as monocytes that circulate through the blood stream are collected and transplanted to adult animals, it is known that the cells do not translocate to the cerebral parenchyma, except when the blood-brain barrier is broken due to external factors (see Non-Patent Document 1).

Thus, the presence of the blood-brain barrier makes it more difficult to achieve an effective concentration of a drug or such, by oral administration or injection, in the brain than in other organs. While an effective drug concentration may be ensured by administering a large dose, this would mean infusing the drug in excess amounts into peripheral blood, which would cause adverse effects such as kidney and liver damage. Therefore, it became necessary to develop a system that selectively transports drugs to the brain. Numerous studies directed to this goal are presently ongoing. Most of such research and development involves efforts to enhance brain localization through chemical modification of the drug itself, by utilizing a property of cerebrovascular endothelial cells - the higher the lipid solubility of a substance, the more easily it passes through the blood-brain barrier. However, such methods improve drug localization in the brain by several folds at best, which is on the whole, no more than an error range. In contrast to peripheral organs where substances permeate through the intercellular spaces of vascular endothelial cells, in the brain, the intercellular spaces of cerebrovascular endothelial cells form special structures called tight junctions and hardly allow permeation of blood components through them. Therefore, transport of substances to the brain requires permeation after chemical modification of the substances to make them lipid-soluble and directly integratable into the cell membrane. More specifically, since there is no alternate route for substance transport to the brain, in contrast to other organs, substances must be made to permeate directly into cells. However, since this mechanism is different from the norm, the efficiency is several thousands to tens of thousands times lower. Accordingly, this method cannot be referred to as brain-specific drug transport.
[Non-patent Document 1] Imai, F., Sawada, M., Suzuki, H. et al. Neurosci Lett 237 1 pp.49-52 (1997)

### Disclosure of the Invention

### [Problems to be Solved by the Invention]

It is conceivable that brain specific transportation of pharmaceutical agents can be achieved by developing carrier molecules that are able to effectively pass through the blood-brain barrier and translocate to brain tissues and by using such carrier molecules to administer pharmaceutical agents, in a form bound to the molecules or encapsulated in the molecules.

The present invention was achieved in view of the above circumstances. An objective of the present invention is to provide carrier molecules that can translocate to the brain.

### [Means for Solving the Problems]

Molecules having the ability to pass through the blood-brain barrier, more particularly peptides having brain-localizing activity, have been previously identified by the present inventors (WO 2005/014625). Using a culture system model of the blood-brain barrier (BBB), the present inventors examined the blood-brain barrier permeating ability of yeast expressing on the cell surface peptides having brain-localizing activity.

As a result, it was discovered that yeasts expressing the peptides having brain-localizing activity on the cell surface permeated the blood-brain barrier. Since this ability to permeate the blood-brain barrier is not observed when such peptides having brain-localizing activity are not expressed on the cell surfaces, the ability to permeate the blood-brain barrier was shown to be a property of the above peptides.

Thus, it was discovered for the first time that even a very large molecule such as yeast can permeate the blood-brain barrier by expressing a brain-localizing polypeptide on its cell surface. One skilled in the art would not have expected that peptides having brain-localizing activity could confer brain-localizing activity even to very large substances such as yeast; accordingly, this is a surprising finding. In fact, even cells other than yeasts can have brain-localizing activity when they express brain-localizing polypeptides on their cell surface.

This finding enables substances expected to have a beneficial effect in the brain to be effectively transported to the brain, for example by encapsulating them in the brain-localizing cells of the present invention.

The brain-localizing cells of the present invention can serve as effective delivery molecules that target the brain tissues. The brain-localizing cells of the present invention find utility as carriers for transport to the brain, to deliver desired compounds to the brain, for example, by intravenous administration.

As described above, the present inventors succeeded in producing cells having brain-localizing activity (brain-localizing cells), discovered that the cells are useful as carriers for translocating desired substances to the brain, and thereby completed the present invention.

The present invention relates to carrier molecules that can translocate to the brain, and more specifically provides the following:
[1] a brain-localizing cell that expresses a polypeptide having brain-localizing activity on the cell surface;
[2] the brain-localizing cell of [1], wherein the polypeptide having brain-localizing activity comprises the following amino acid motif sequence:
   X₁-(R or K)-X₃-X4, or
   X₄-X₃-(R or K)-X₁,
   wherein X₁ denotes S, T, N, P, V, or L; X₃ denotes an arbitrary amino acid; and X₄ denotes G, S, T, C, N, L, Q, or Y;
[3] the brain-localizing cell of [2], wherein the amino acid motif sequence is the following sequence:
   X₁-(R or K)-X₃-X₄, or
   X₄-X₃-(R or K)-X₁,
   wherein X₁ denotes S, T, N, P, or V; X₃ denotes an arbitrary amino acid; and X₄ denotes an uncharged polar amino acid (G, S, T, C, N, Q, or Y);
[4] the brain-localizing cell of [2], wherein the amino acid motif sequence is the following sequence:
   X₁-(R or K)-X₃-X₄, or
   X₄-X₃-(R or K)-X₁,
   wherein X₁ denotes S, T, P, or L; X₃ denotes an arbitrary amino acid; and X₄ denotes S, T, C, L, or Q;
[5] the brain-localizing cell of [1], wherein the polypeptide having brain-localizing activity comprises any one of the amino acid sequences of SEQ ID NOs: 1 to 12;
[6] the brain-localizing cell of any one of [1] to [5], wherein the length of the polypeptide having brain-localizing activity is 9 amino acids or shorter;
[7] the brain-localizing cell of any one of [1] to [6], wherein the cell has the ability to permeate the blood-brain barrier;
[8] the brain-localizing cell of any one of [1] to [7], wherein the cell is a yeast cell;
[9] a carrier for delivery to the brain which comprises as an active ingredient the brain-localizing cell of any one of [1] to [8];
[10] a brain-localizing pharmaceutical agent comprising the carrier for delivery to the brain of [9] which comprises a biologically active substance;
[11] the brain-localizing pharmaceutical agent of [10], wherein the biologically active substance has a therapeutic effect on a brain disease;
[12] a method for producing a brain-localizing cell, which comprises the step of expressing a polypeptide having brain-localizing activity on the cell surface;
[13] the method of [12], which comprises the step of introducing a nucleic acid encoding the polypeptide having brain-localizing activity into the cell;
[14] a method for producing a brain-localizing pharmaceutical agent, wherein the method comprises the step of introducing a biologically active substance into the brain-localizing cell of any one of [1] to [8];
[15] a kit for producing a brain-localizing cell, wherein the kit comprises a cell and a nucleic acid encoding a polypeptide having brain-localizing activity as components; and
[16] a method for translocating a desired substance to the brain of a non-human animal, wherein the method comprises the following steps (a) and (b):
   (a) introducing a desired substance into the brain-localizing cell of any one of [1] to [8] to produce a brain-localizing cell comprising the substance, and
   (b) administering the brain-localizing cell produced in step (a) to the body of a non-human animal.

   Furthermore, the present invention relates to the following:
[17] a method for treating a brain disease, wherein the method comprises the step of administering a brain-localizing cell, a carrier for delivery to the brain, or a brain-localizing pharmaceutical agent to an individual (a patient or such); and
[18] use of a bone marrow progenitor cell or a carrier for delivery to the brain for producing a brain-localizing pharmaceutical agent (a therapeutic agent for a brain disease).

### Brief Description of the Drawings

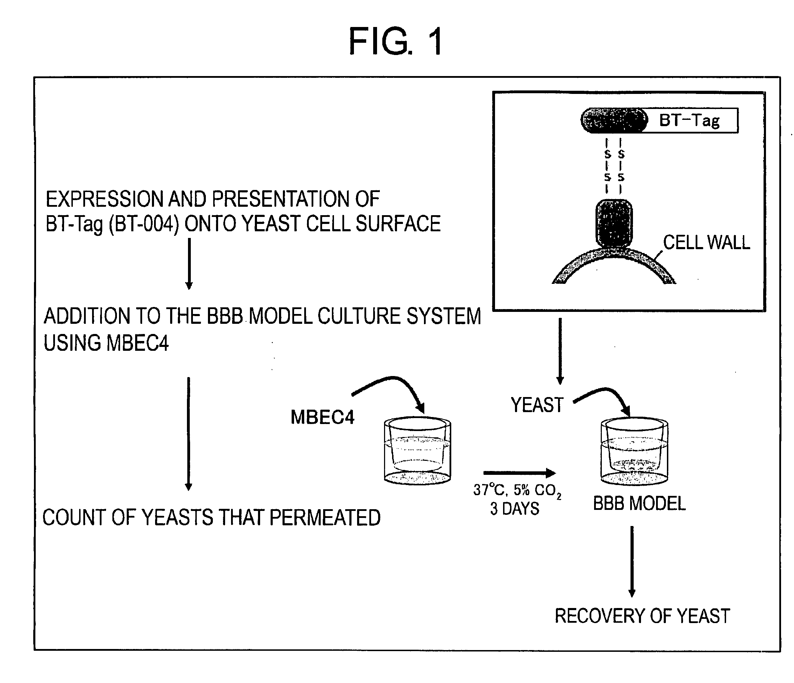

Fig. 1 is an outline flow diagram representing confirmation of the screening with yeast.
Fig. 2 is composed of a set of photographs depicting the expression of BT 004V3L peptide on yeast cell surface. Left: T2J004V3LYEAST strain expressing BT-004V3L. Right: wild-type strain EBY-100 not expressing BT-004V3L (EBY-100 strain without the introduction of the vector). Bright images in the EBY-100 strain into which the vector was not introduced (Fig. 2, right) represent the background signal.
Fig. 3 is composed of a set of photographs and a diagram comparing the respective numbers of permeated yeasts of T2J004V3LYEAST and control strains that were added to the BBB model of MBEC4.

### Best Mode for Carrying Out the Invention

The present inventors discovered that cells expressing polypeptides having brain-localizing activity on the cell surface have the activity to translocate to the brain (brain-localizing activity). These cells having brain-localizing activity (hereafter, such cells are referred to as "brain-localizing cells" or simply as "cells of the present invention") are useful as carriers for translocating (transporting) desired substances to the brain.

The present invention provides brain-localizing cells that express peptides having brain-localizing activity on the cell surface.

The cells of the present invention have the activity to permeate (pass through) the blood-brain barrier.

In general, translocation of substances from blood to brain tissues is restricted by a structure referred to as the blood-brain barrier (BBB). This structure protects the brain from harmful substances and such. In the context of the present invention, the term "brain-localizing activity" refers to an activity of a cell to translocate to the brain tissues when the cell is administered (for example, by intravenous administration) into the body of an organism (mammal). The cells of the present invention can be generally referred to as cells having brain-localizing activity (brain-localizing cells), but can also be referred to as, for example, cells having the activity to pass through the blood-brain barrier.

The term "translocation to the brain" in the context of the present invention refers more specifically to translocation to the brain parenchyma or the brain tissues. Furthermore, the above-mentioned passage through the blood-brain barrier is presumed to arise from transmigration. Therefore, the cells of the present invention may be referred to as, for example, cells having the activity to induce transmigration (transcytosis); however, they need not necessarily be construed as limited thereto.

The term "transmigration" above refers to a phenomenon in which a certain molecule penetrates into the brain by passing through a vascular endothelial cell rather than the intercellular space between vascular endothelial cells. This is also called "trans-endothelial cell migration", "transcellular pathway", or "transcytosis". Molecules (cells and such) that pass through vascular endothelial cells by this mechanism may present signal molecules on their surface and thereby induce the above-mentioned phenomenon in vascular endothelial cells via receptors on the surface of the vascular endothelial cells.

In the context of the present invention, the types of "cells" are not particularly limited. For example, so long as cells are approximately as large as yeast, they can be effectively delivered to the brain by expressing the polypeptides having brain-localizing activity of the present invention on their cell surface. Even cells larger than yeast can be delivered to the brain if they are in the size range that can pass through the blood-brain barrier by the aforementioned transmigration phenomenon. Therefore, the cells of the present invention are not particularly limited, and may be exemplified, specifically, by mammalian (human, monkey, mouse, rat or such) cells, eukaryotic cells (nematodes, yeasts, fungi or such), or prokaryotic cells *(Escherichia coli, Bacillus subtilis* or such). The size of the cells of the present invention is not particularly limited; however, they are preferably of a size equivalent to or smaller than yeast.

The brain-localizing cells of the present invention can be used as carriers for translocating desired substances (compounds) into brain neurons. The desired compounds can be generally translocated to the brain by using the cells of the present invention to support them.

In the present invention, the term "to support" means to introduce a desired compound into a cell of the present invention, or to bind or adsorb the same to the surface of a cell of the present invention. In addition, a desired protein can be delivered to the brain in a manner in which the protein is expressed on the surface of a brain-localizing cell of the present invention.

One skilled in the art can use the brain-localizing cells of the present invention to support desired compounds by applying generally-known techniques. For example, desired compounds can be introduced into cells by the calcium phosphate method, electrical pulse, lipofection, aggregation, microinjection, particle gun, DEAE-dextran method, electroporation, lipofection or such.

Compounds which can be introduced into cells of the present invention are not particularly limited, and can include proteins, nucleic acids (expression vectors, anti-sense RNAs, siRNAs and such), and sugar chains. For example, desired proteins can be introduced into cells of the present invention by introducing DNAs encoding the proteins or expression vectors containing the DNAs.

The present inventors discovered that 9-amino acid polypeptides can confer effective brain-localizing activity to other substances (molecules). Therefore, it is apparent that, at the least, polypeptides as short as 9 amino acids can confer effective brain-localizing activity to other molecules.

In the context of the present invention, the length of "polypeptides having brain-localizing activity" is not particularly limited, but is, for example, 100 amino acids or shorter, preferably 15 amino acids or shorter, more preferably 9 amino acids or shorter, and most preferably 4 to 9 amino acids.

Polypeptides containing one of the sequences listed below were discovered by the present inventors to have brain-localizing activity (WO 2005/014625). The polypeptides having brain-localizing activity of the present invention are specifically exemplified by the following peptides, but are not particularly limited thereto.

**[Table 1]**

| | |
|---|---|
| **Name** | **Amino Acid Sequence** |
| T2J001 | CSNLLSRHC (SEQ ID NO: 1) |
| T2J002 | CSLNTRSQC (SEQ ID NO: 2) |
| T2J003 | CVAPSRATC (SEQ ID NO: 3) |
| T2J004 | CVVRHLQQC (SEQ ID NO: 4) |
| T2J004V3L | CVLRHLQQC (SEQ ID NO: 5) |
| T2J006 | CRQLVQVHC (SEQ ID NO: 6) |
| T2J007 | CGPLKTSAC (SEQ ID NO: 7) |
| T2J008 | CLKPGPKHC (SEQ ID NO: 8) |
| T2J009 | CRSPQPAVC (SEQ ID NO: 9) |
| T2J012 | CNPLSPRSC (SEQ ID NO: 10) |
| T2J013 | CPAGAVKSC (SEQ ID NO: 11) |
| T2J013V6L | CPAGALKSC (SEQ ID NO: 12) |

When a polypeptide arbitrarily selected from the above peptides (SEQ ID NO: 5) was expressed on yeast cell surface, the yeast was shown to have the ability to permeate the blood-brain barrier (see Examples described later). Thus, the present inventors discovered for the first time that the polypeptides of the present invention having brain-localizing activity can confer brain-localizing activity even to very large molecules, such as yeast.

Furthermore, even polypeptides other than the above which, for example, have amino acid sequences with one or several (for example, one to three, preferably one to two, and more preferably one) amino acid additions, deletions, or substitutions in any one of SEQ ID NOs: 1 to 12 and have brain-localizing activity, are included as polypeptides having brain-localizing activity of the present invention.

Naturally-occurring amino acid residues are generally classified, according to the property of their side chains, into the following groups: (1) hydrophobic amino acids: alanine, isoleucine, norleucine, valine, methionine, and leucine; (2) neutral amino acids: asparagine, glutamine, cysteine, threonine, and serine; (3) acidic amino acids: aspartic acid and glutamic acid; (4) basic amino acids: arginine, histidine, and lysine; (5) amino acid residues that have an effect on the orientation of chains: glycine and proline; and (6) aromatic amino acids: tyrosine, tryptophan, and phenylalanine. Therefore, when the alteration of an amino acid is carried out in the context of the present invention, it is not particularly limited; however, an amino acid is preferably altered to another amino acid having a similar property as mentioned above.

Whether a desired polypeptide has brain-localizing activity can be evaluated, for example, with the BBB model using MBEC4 described below. Therefore, when amino acids in the above polypeptides of SEQ ID NOs: 1 to 12 are altered, it is within ordinary trial and error for one skilled in the art to select polypeptides having brain-localizing activity from among the altered polypeptides.

In a preferred embodiment of the present invention, polypeptides having brain-localizing activity include the amino acid motif sequence described in [Sequence 1] below, more preferably the amino acid motif sequence of [Sequence 2], or the amino acid motif sequence of [Sequence 3].
[Sequence 1]
   X₁-(R or K)-X₃-X₄, or
   X₄-X₃-(R or K)-X₁
      wherein X₁ denotes S (serine), T (threonine), N (asparagine), P (proline), V (valine), or L (leucine);
   X₃ denotes an arbitrary amino acid;
   X₄ denotes G (glycine), S (serine), T (tyrosine), C (cysteine), N (asparagine), L (leucine), Q (glutamine), or Y (tyrosine).
[Sequence 2]
   X₁-(R or K)-X₃-X₄, or
   X₄-X₃-(R or K)-X₁
      wherein X₁ denotes S (serine), T (threonine), N (asparagine), P (proline), or V (valine) [however, more preferably, X₁ denotes S or T];
   X₃ denotes an arbitrary amino acid;
   X₄ denotes G (glycine), S (serine), T (tyrosine), C (cysteine), N (asparagine), Q (glutamine), or Y (tyrosine) [however, more preferably, X₄ denotes T, Q, or C].
      Furthermore, in the above-mentioned (R or K), R is more preferred.
      The amino acids G, S, T, C, N, Q, and Y are generally classified as uncharged polar amino acids.
[Sequence 3]
   X₁-(R or K)-X₃-X₄, or
   X₄-X₃-(R or K)-X₁
      wherein X₁ denotes S (serine), T (threonine), P (proline), or L (leucine);
   X₃ denotes an arbitrary amino acid;
   X₄ denotes G (glycine), S (serine), T (tyrosine), C (cysteine), L (leucine), or Q (glutamine).

Herein, amino acids are represented by a commonly used one letter notation (for example, R stands for arginine and K stands for lysine). In addition, amino acid sequences are shown in a commonly used direction, from the N-terminus to the C-terminus.

Furthermore, a polypeptide having a cyclic structure (more specifically, a structure in which a disulfide bond (S-S bond) is formed between terminal cysteine residues of the above peptide and the peptide is cyclized) is expected to have even higher brain-localizing activity. Therefore, in a preferred embodiment of the present invention, polypeptides having brain-localizing activity have a cyclic structure. Polypeptides constituting the cyclic region preferably contain the motif sequences described above. The amino acids in the motif sequences are composed of four contiguous amino acid residues. These contiguous amino acids usually form peptide bonds between each other, though they can form a disulfide bond if they are cysteine residues. The above-mentioned "four contiguous amino acid residues" in the motif sequences of the present invention are not limited to those forming peptide bonds, but also include those that form disulfide bonds when the contiguous amino acids are cysteine residues. Specifically, although the notation "-" used in the above-mentioned [Sequence 1] to [Sequence 3] usually refers to a peptide bond, it may refer to a disulfide bond (S-S bond) when the contiguous amino acids are cysteine residues. For example, even if the above motif sequences are not found in an amino acid sequence of straight chain polypeptides, the motifs may be formed by amino acids that are neighbored by the formation of a cyclic structure.

In a preferred embodiment of the present invention, so long as polypeptides having brain-localizing activity contain the above-mentioned motif sequences composed of four amino acids, they are not particularly limited in terms of the type of amino acid sequence other than the motif sequences.

The polypeptides having brain-localizing activity as listed in Table 1 have the following characteristics.

In the polypeptide regions that can form a cyclic structure (i.e., the amino acid sequences excepting cysteine residues at both ends), (1) all polypeptides contain a basic amino acid, K or R, and (2) the remaining amino acid residues are composed of any of 10 amino acids [G, A, V, L, S, T, P, Q, H, and N].

Thus, in a preferred embodiment of the present invention, polypeptides having brain-localizing activity have a cyclic region and at least one or more basic amino acid residues (K or R) in the cyclic region, wherein the remaining amino acid residues in the cyclic region are selected from amino acid residues [G, A, V, L, S, T, P, Q, H, and N] (usually 80% or more, preferably 85% or more, more preferably 90% or more, even more preferably 95% or more, and most preferably 100%) (this characteristic may be referred to herein as "Feature 1"). In a more preferred embodiment of the present invention, polypeptides having brain-localizing activity have the above "Feature 1" and contain the motif sequences of the present invention ([Sequence 1] to [Sequence 3]).

As described in the above-mentioned "Feature 1", all of the polypeptides having brain-localizing activity of the present invention were found to contain a basic amino acid (K or R). For example, the polypeptides composed of nine amino acids described above were shown to contain at least one basic amino acid (the content in all amino acids: 1/9 = 0.11 (11 %) or more).

Therefore, an embodiment of the polypeptides having brain-localizing activity of the present invention can include the following polypeptides:
(a) polypeptides having brain-localizing activity in which basic amino acids (K or R) make up 10% or more of the polypeptides;
(b) polypeptides having brain-localizing activity that have a cyclic peptide region, in which basic amino acids (K or R) make up 10% or more of the cyclic peptide region; and
(c) polypeptides having brain-localizing activity that have a cyclic peptide region and contain at least one or more basic amino acids (K or R) in the cyclic peptide region.

The upper limit of the length of the polypeptides having brain-localizing activity of the present invention is not particularly limited. Typically, polypeptides that are 7 amino acids or longer and contain a 4 amino acid sequence motif or the aforementioned "Feature 1" are considered to have effective brain-localizing activity. Although there is no upper limit on the length, polypeptides with a length of 50 amino acids or shorter, or preferably 35 amino acids or shorter, are generally considered to have a sufficient brain-localizing activity. Furthermore, since even long polypeptides containing these polypeptides generally possess brain-localizing activity as well, the length of the polypeptides having brain-localizing activity of the present invention is not limited.

In a preferred embodiment of the present invention, the length of a polypeptide region to be cyclized in polypeptides having brain-localizing activity is not particularly limited. Although the length of a cyclic peptide region in the polypeptides is not particularly limited, it may be, for example, 100 amino acids or shorter, preferably 50 amino acids or shorter, more preferably 4 to 30 amino acids, even more preferably 4 to 15 amino acids, yet even more preferably 4 to 9 amino acids, and most preferably 4 to 7 amino acids.

Brain-localizing cells of the present invention have the activity to permeate the blood-brain barrier. Therefore, the cells of the present invention can be distinguished from other cells by, for example, using as an index their ability to permeate the blood-brain barrier.

Whether a desired cell has the ability to permeate the blood-brain barrier can be suitably determined by utilizing, for example, a blood-brain barrier model using mouse brain capillary endothelial cell, MBEC4. More specifically, whether a desired cell has the ability to permeate the blood-brain barrier can be evaluated by the method described in Examples below or by the methods with appropriate modifications. Accordingly, the cells of the present invention can be appropriately selected from a heterogeneous population containing a variety of cells.

Whether an arbitrary cell has the brain-localizing activity of the present invention can be examined by using, for example, experimental animals and such. Specifically, it can be evaluated by introducing a marker gene (for example, GFP) into a test cell, administering the cell in the vein (for example, the tail vein) of a test animal such as mouse, and then detecting the presence or absence of the marker gene in the animal's brain. If the cell administered in the vein reaches the brain (for example, if the cell is detected in the brain), the cell is judged to have the brain-localizing activity of the present invention.

A polypeptide having brain-localizing activity of the present invention can be, for example, any one of the following polypeptides (a) to (c):
(a) a polypeptide having any one of the amino acid sequences of SEQ ID NOs: 1 to 12;
(b) a polypeptide having a cyclic peptide region formed by a disulfide bond between terminal cysteine residues of the polypeptide of (a) above; and
(c) a polypeptide having brain-localizing activity which comprises an amino acid sequence with one or several amino acid additions, deletions, or substitutions in any one of SEQ ID NOs: 1 to 12.

For example, yeasts may be suitably used as cells of the present invention. Types of yeasts are not particularly limited, and can include, for example, *Saccharomyces cerevisiae.*

A desired substance (compound) can be transported to the brain by utilizing the brain-localizing cells of the present invention. Specifically, a desired substance can be translocated to the brain by introducing the substance into a brain-localizing cell of the present invention or by binding it to such a cell.

Accordingly, the present invention provides carriers (carrier molecules) for delivery to the brain that comprise brain-localizing cells of the present invention. The carriers of the present invention may be referred to as, for example, carriers, transporters, vectors, or delivery molecules.

Substances to be supported by the carriers for delivery to the brain of the present invention are not particularly limited; however, in general, they are preferably biologically active substances. Biologically active substances include, for example, substances having an activity in the brain, and preferably, are substances having therapeutic or prophylactic effects on brain diseases (for example, cranial nerve diseases). One embodiment of such substances can suitably include nucleic acids such as DNAs (including vectors) or RNAs (including anti-sense RNAs and siRNAs). For example, a DNA encoding a protein having a therapeutic effect on a brain disease is one example of the above-mentioned biologically active substances.

Desired pharmaceutical agents can be translocated to the brain using the carriers of the present invention. For example, by using the carriers to support (encapsulate) compounds (pharmaceutical compositions) that have therapeutic effects on brain diseases, the compounds can be delivered efficiently to the brain and can exert an effective therapeutic effect. The carriers used to support such compounds (pharmaceutical compositions) themselves are expected to serve as therapeutic agents for brain diseases. Accordingly, the present invention provides therapeutic agents for brain diseases having a structure in which the carriers for delivery to the brain of the present invention are used to support drugs.

Herein, the term "to support" may refer to introducing (encapsulating) a drug into a cell of the present invention, i.e., a carrier molecule; directly binding (adsorbing) the drug to the surface of a cell of the present invention; or mixing the drug with a carrier molecule. For example, a drug may be encapsulated into a cell of the present invention using a liposome or microcapsule and such, or, when a drug is a protein, an expression vector carrying a DNA encoding the protein in an expressible state may be introduced into a cell of the present invention. A desired drug may also be encapsulated in a liposome or microcapsule and such, and the liposome or microcapsule may be bound to a carrier molecule (for example, a cell surface) of the present invention.

The substances that can be delivered to the brain by the brain-localizing cells (carriers) of the present invention may include a variety of substances, depending on the type of brain disease and disorder. For example, they can include not only neurotrophic factors, such as BDNF and GDNF, and inhibitory cytokines such as IL-10 and TGFβ but also drugs such as dopamine for treating Parkinson's disease and donepezil hydrochloride for treating Alzheimer's disease. Many of drugs are barely translocated to the brain without drug modification (i.e., they are degraded before they reach the brain), as shown in levodopa, a dopamine prodrug. Accordingly, drugs which show efficacy *in vitro* but not *in vivo* may become feasible by using the cells of the present invention. In addition, drugs which are often used today, such as donepezil hydrochloride, a therapeutic drug for Alzheimer type dementia, can now be used at a reduced dose by directly tranlocating to the brain.

Brain-localizing cells of the present invention can be applied, for example, to the following four cases as a therapeutic strategy for treating brain diseases:
1) Replacement therapies that supplement enzymes and bioactive proteins whose amounts and activities have decreased due to deletions or mutations: These therapies are performed for various brain diseases caused by deficiencies of particular enzymes or proteins in the brain due to genetic deletions or mutations, by injecting cells into which genes encoding the deficient proteins or enzymes have been introduced. For degenerative loss of particular neurons in Parkinson's disease and Alzheimer's disease, genes that promote synthesis of neurotransmitters which become deficient due to the degenerative loss of nerves may be used; for example, genes of enzymes involved in dopamine biosynthesis including tyrosine hydroxylase and biopterin synthase may be used for Parkinson's disease.
2) Protective therapies for protecting neurons that would otherwise be lost by degeneration or such and for strengthening their functions: These therapies can be performed by injecting cells expressing genes of neurotrophic factors, such as NGF, BDNF, GDNF, and NT3 (BDNF and GDNF are known to be particularly effective for Parkinson's disease), which suppress neuron death by various causes, including degenerative diseases and cerebral ischemia, and promote the regeneration of neurites. Furthermore, therapies for diseases that involve immune cells, such as multiple sclerosis, can be performed by introducing the TGF-β gene or IL-10 gene, which have immunosuppressive effects, into the cells of the present invention.
3) Methods for removing tumors, blood clots and such: These methods can be performed by expressing factors that have antitumor effects, or by transferring the cells of the present invention that carry an antitumor agent into the brain. For removal of blood clots, fibrinolytic enzymes can be expressed.
4) Methods for introducing effective drugs exclusively into the brain: Among drugs that act on the nervous system, some have high peripheral toxicity, some act on the peripheral nervous system, and others cannot easily pass through the blood-brain barrier; therefore, drug delivery systems that are specific for the brain are necessary. Using the cells of the present invention, drugs may be delivered specifically to the brain, with little effect on peripheral organs.

Diseases that can be treated using brain-localizing cells of the present invention are not particularly limited, and can include, for example, Sandhoff's disease and Gaucher's disease. These diseases are also called lysosomal diseases because enzymes and proteins in lysosomes are deficient and thereby cause the accumulation in lysosomes of substances that should be normally metabolized, which, in turn, induces various symptoms. At present, the primary method for treating these diseases is an enzyme replacement therapy in which an enzyme is injected in the blood stream to ameliorate the symptoms. If brain-localizing cells of the present invention can be used for lysosomal diseases, a cell therapy may be performed, without using the cells to transport drugs or genes, because the cells can normally produce the enzymes and can supplement the role of cells deficient in the enzymes.

When the cells of the present invention are used to transport genes and drugs, they can be applied to various diseases, such as brain tumors, brain ischemia, Parkinson's disease, and Alzheimer's disease. In the case of brain ischemia, local neuroprotection can be promoted using neurotrophic factors such as BDNF or erythropoietin; and in the case of a chronic disorder, Parkinson's disease, local replacement of dopamine can be performed. Furthermore, in the treatment of brain tumors, side effects are serious issues not only because drugs fail to translocate to the tumor sites but also because most of anticancer agents affect proliferative cells (due to lack of cancer selectivity); however, these problems can also be circumvented by using the present invention.

As described above, the present invention provides brain-localizing pharmaceutical agents (brain-localizing pharmaceutical compositions) which comprise the brain-localizing cells (carriers for delivery to the brain) of the present invention comprising biologically active substances. The biologically active substances are typically substances (compounds) that have a therapeutic or prophylactic effect on brain disease.

The pharmaceutical agents of the present invention can be formulated using known pharmaceutical production methods. For example, the pharmaceutical agents can be formulated into pharmaceutical formulations suitable for effective administration into the body, such as in the form of injections, transnasal formulations, transdermal formulations, and oral agents, though preferably injections, by suitably combining the agent with appropriate conventionally used carriers or vehicles such as sterilized water, physiological saline, vegetable oil (for example, sesame oil and olive oil), coloring agent, emulsifier (for example, cholesterol), suspending agent (for example, gum arabic), surfactant (for example, polyoxyethylene hardened castor oil surfactants), solubilizing agent (for example, sodium phosphate), stabilizer (for example, sugars, sugar alcohols, and albumin), or preservative (for example, paraben). For example, injection formulations can be provided as freeze-dried products, solutions for injections, or such.

Furthermore, administration into the body can be carried out by a method known to one skilled in the art, for example, by intraarterial injection, intravenous injection, or subcutaneous injection, or intranasally, transbronchially, intramuscularly, or orally; however, intraarterial and intravenous administrations are preferable. The dose may vary depending on the age, body weight, and symptoms of the patient, administration method and such. However, one skilled in the art can appropriately select a suitable dose.

The present invention relates to methods for producing the brain-localizing cells of the present invention. In a preferred embodiment of the present invention, the methods include the step of expressing polypeptides having brain-localizing activity on cell surfaces.

Expression (presentation) of an arbitrary polypeptide on the cell surface of a microorganism can be performed by those skilled in the art based on a known technique or knowledge. For example, the expression of arbitrary polypeptides on cell surfaces can be carried out by utilizing a commercially available kit (Invitrogen, Catalog no. V835-01, "pYD1 Yeast Display Vector Kit"). For example, expression of the polypeptide on the cell surface of a microorganism can be achieved by producing DNA vectors which can express fusion proteins of polypeptides having brain-localizing activity and proteins to be expressed on cell surfaces, and then introducing the vectors into cells. Those skilled in the art can appropriately produce the DNA vectors using conventional genetic engineering techniques.

Accordingly, in a preferred embodiment of the present invention, methods for producing cells comprise the step of expressing (presenting) polypeptides having brain-localizing activity on cell surfaces. More specifically, the methods comprise the step of introducing nucleic acids encoding polypeptides having brain-localizing activity into cells.

In addition, the present invention relates to methods for producing brain-localizing cells that comprise the above-mentioned biologically active substances. In a preferred embodiment, the production methods comprise the step of introducing or binding the biologically active substances to the carriers of the present invention.

These methods more specifically comprise the steps (a) expressing polypeptides having brain-localizing activity on cell surfaces to produce brain-localizing cells, and (b) introducing biologically active substances into the brain-localizing cells of step (a).

Brain-localizing cells of the present invention can also be produced by directly binding peptides having brain-localizing activity to the cell surface. This method is also encompassed by the present invention. In a preferred embodiment of the present invention, the methods for producing brain-localizing cells comprise the step of contacting peptides having brain-localizing activity to cells.

The present invention provides kits for preparing brain-localizing cells of the present invention and kits for producing brain-localizing cells that comprise the biologically active substances of the present invention.

The kits of the present invention can comprise as components, for example, cells, polypeptides having brain-localizing activity, media for cell culture, and culture solutions.

In a preferred embodiment of the present invention, kits for preparing brain-localizing cells comprise, at a minimum, cells and polypeptides having brain-localizing activity as components.

The kits of the present invention can comprise the brain-localizing cells of the present invention as a sample (control).

The kits for producing brain-localizing cells that comprise biologically active substances of the present invention may comprise as components brain-localizing cells of the present invention, media for cell culture, and reagents for introducing the biologically active substances into the cells. The reagents can include, for example, various reagents for transfecting cells.

In a preferred embodiment of the present invention, the kits for producing brain-localizing cells that comprise biologically active substances comprise, for example, at a minimum, the following (a) as a component, and more preferably comprise at a minimum the following (a) and (b) as components:
(a) brain-localizing cells of the present invention,
(b) media for culturing brain-localizing cells of the present invention (culture solutions and such).

In addition to the above components, biologically active substances to be introduced into the cells of the present invention can be packaged in the kits of the present invention.

As the above-mentioned "media", those commonly used for cell culture can be used. Those skilled in the art can easily find the basic composition and such of the "media" in literature, manuals and such.

The kits of the present invention can comprise various reagents for use in various methods for separating cells of the present invention, for example, the BBB model of MBEC4. Furthermore, specifications of the kits of the present invention, instruction manuals of the methods and such can be suitably packaged in the kits.

The present invention relates to methods for translocating desired substances (for example, vectors and such that can express DNAs encoding biologically active proteins) to the brain of animals using brain-localizing cells of the present invention. The methods preferably comprise the following steps (a) and (b):
(a) introducing desired substances to be translocated to the brain, into brain-localizing cells of the present invention, to produce brain-localizing cells comprising the substances; and
(b) administering the brain-localizing cells produced in step (a) to the body of animals.

In the above methods, the term "the body" usually refers to sites other than the brain. For example, desired substances can be translocated to the brain by intravenously administering brain-localizing cells of the present invention to animals.

In the above methods, the term "animal" is not particularly limited, so long as it has a blood-brain barrier; however, it typically includes humans and non-human animals such as monkeys, mice, dogs, and cats.

The present invention provides compositions comprising pharmaceutically acceptable carriers in addition to brain-localizing cells of the present invention, carriers for delivery to the brain of the present invention, or brain-localizing pharmaceutical agents of the present invention.

Furthermore, the present invention relates to methods for treating brain diseases comprising the step of administering to individuals (patients and such) brain-localizing cells of the present invention, carriers for delivery to the brain of the present invention, or brain-localizing pharmaceutical agents of the present invention.

Administration to an individual can be generally carried out by conventional methods known to those skilled in the art, for example, by intraarterial injection, intravenous injection, and subcutaneous injection. The dose will vary depending on the body weight and age of the patient, administration method and such; however, those skilled in the art (physicians, veterinarians, pharmacists and such) can appropriately select a suitable dose.

Furthermore, the present invention relates to uses of brain-localizing cells of the present invention or carriers for delivery to the brain of the present invention for producing brain-localizing pharmaceutical agents (therapeutic agents for brain diseases).

All prior art references cited herein are incorporated herein by reference.

### Examples

Herein below, the present invention will be specifically described with reference to Examples; however, it should not to be construed as being limited thereto.

### [Example 1] Construction of a vector containing a DNA encoding a polypeptide having brain-localizing activity

The primers listed below were prepared based on the amino acid sequence of T2J004V3L (BT004) (CVLRHLQQC; SEQ ID NO: 5), which was identified by the present inventors as a peptide having brain-localizing activity. T2J004V3L-Fw primer:
GGTACCTGCGTCTTGAGACATCTACAACAATGTTAA (SEQ ID NO: 13) T2J004V3L-Rv primer:
CTCGAGTTAACATTGTTGTAGATGTCTCAAGACGCA (SEQ ID NO: 14)

These primers, T2J004V3L-Fw and T2J004V3L-Rv, were heated for 3 minutes at 98°C, annealed by cooling slowly, and then treated with restriction enzymes, KpnI and XhoI, to produce a KpnI cohesive end at one side and an XhoI cohesive end at another side.

After pyD 1 vector (pYD 1 yeast display vector; Invitrogen Co. Ltd., USA) was cleaved by restriction enzymes, KpnI and XhoI, the above-mentioned annealed sequence was introduced into the KpnI-XhoI site of the pyD 1 vector using T4 DNA ligase to produce a peptide expression vector, pyD-T2J004V3L. Whether the introduction was correctly performed was confirmed by DNA sequencing.

### [Example 2] Introduction into yeast

*Saccharomyces cerevisiae* EBY100 strain (Invitrogen Co. Ltd., USA) was used as yeast. The peptide expression vector pyD-T2J004V3L, prepared in Example 1, was introduced into EBY100 strain by transformation. The introduction was performed according to the method of Gietz et al. (Transformation of yeast by lithium acetate/single-stranded carrier DNA/polyethylene glycol method. Methods Enzymol. (2002) 350: 87-96.; High-efficiency transformation of plasmid DNA into yeast. Methods Mol Biol. (2001) 177: 85-97).

Yeast cells in which expression was confirmed were selected from the transformed strain and named T2J004V3LYEAST. A control strain expressing pyD1 vector alone was also prepared. These strains were used for a permeation experiment using the BBB model of MBEC4, described in Example 4 below.

### [Example 3] Induction of expression in yeast

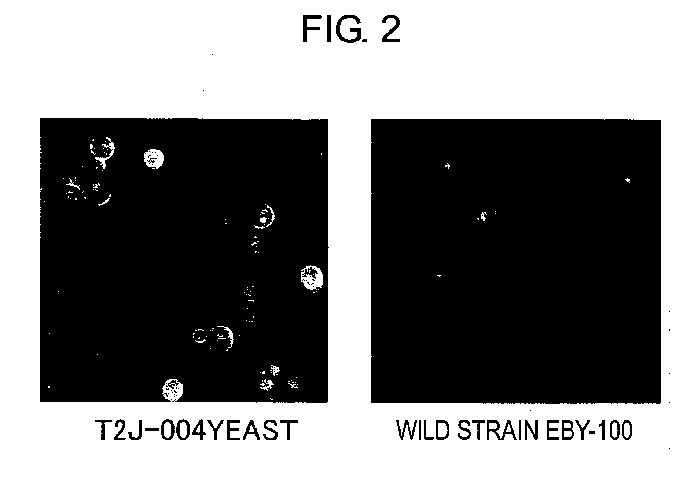

Prior to induction, preculture of T2J004V3LYEAST and the control strain was performed by culturing them overnight at 30°C in YNB-CAA, 2% glucose liquid medium (0.17% amino acid, ammonium sulfate-free Yeast nitrogen base (BD Difco Co. Ltd., USA), 0.5% ammonium sulfate (Wako Co. Ltd., Japan), 0.5% casamino acid (Nippon seiyaku Co. Ltd., Japan), and 2% glucose). After the overnight culture, yeasts were washed in physiological saline, seeded in YNB-CAA, 2% galactose liquid medium (0.17% amino acid, ammonium sulfate-free Yeast nitrogen base (BD Difco Co. Ltd., USA), 0.5% ammonium sulfate (Wako Co. Ltd., Japan), 0.5% casamino acid (Nippon seiyaku Co. Ltd., Japan), and 2% galactose) so that the OD value reached 0.5 to 1.0 (representing 0.5 x 10⁷ to 1.0 x 10⁷ cells/ml), and then cultured for 24 to 36 hours at 20°C. By culturing in the YNB-CAA, 2% galactose medium, expression of BT-tag (hereafter, referred to as BT-004V3L), a polypeptide having brain-localizing activity that was fused with the Aga2 protein, was induced. The polypeptide was presented on the yeast cell surface by binding to Agal, which was localized on the cell surface, by a disulfide bond. In the control, only the Aga2 protein was induced to express and presented on the yeast cell surface. The EBY-100 strain without the introduction of vectors shown on the right in Fig. 2 indicates no presentation on the cell surface. (Fig. 2)

### [Example 4] Construction of a blood-brain barrier (BBB) model using a mouse brain capillary endothelial cell, MBEC4

A BBB model was constructed as follows: Cell culture inserts for 6-well or 12-well plates with a pore size of 8.0 µm (BD Falcon Co. Ltd., USA) were used. In addition, cell culture insert companion plates for 6-well or 12-well plates (BD Falcon Co. Ltd., USA) were used.

Nylon membranes of the cell culture inserts were coated with 10-fold diluted collagen I-A (Nitta Gelatin) to form thin membranes of collagen. Onto the membranes, 1.0 ml (6-well) or 0.25 ml (12-well) of an MBEC4 cell culture (MBEC Medium) in which the cells were suspended at 5.0 x 10⁴ cells/ml in MBEC Medium (Dulbecco's Modified Eagle's Medium (DMEM), 10% Fatal bovine serum (FBS; BD GIBCO Co. Ltd., USA), 2.7 µg/ml Endothelial Cell Growth Supplements (ECGS; Wako Co. Ltd., Japan), and 0.2% 25 mg/ml Heparin (Sigma Co. Ltd., USA)), was added and then cultured at 37°C in the presence of 5% CO₂. After 12 hours of culture, 2.0 ml (6-well) or 1.0 ml (12-well) of the MBEC Medium was further added and cultured. After an additional 48 hours of culture, the MBEC Medium of the upper chamber was adjusted to 1.0 ml (6-well) or 0.5 ml (12-well), and 2.5 ml (6-well) or 1.5 ml (12-well) of the MBEC Medium was newly added to the lower chamber, and subsequently the cells were cultured for 12 hours.

2.5 x 10⁷ cells (6-well) or 1.5 x 10⁷ cells (12-well) of yeast (T2J004V3LYEAST strain or the control strain) were added to the upper chamber and incubated for 4 to 6 hours at 37°C in the presence of 5% CO₂. After the incubation, the medium in the lower chamber was collected, centrifuged for 5 minutes at 12,000 rpm to remove the supernatant, and then suspended in 200 µl of physiological saline. The total volume of cell suspension was applied to YPD agar medium (5% YPD medium (BD Difco Co. Ltd., USA) and 1.5% agar (Wako Co. Ltd., Japan) for cell culture. The agar medium was incubated for 36 to 48 hours at 30°C and then colonies were counted.

The above permeation experiment was performed 16 times for T2J004V3LYEAST strain expressing BT-004V3L and 17 times for the control strain.

As a result, the average numbers of yeasts that permeated were 176 and 37 for T2J004V3LYEAST strain and the control strain, respectively. When one each of the highest and lowest numbers were left out, the average numbers were 121 and 29 for T2J004V3LYEAST strain and the control strain, respectively. These results are summarized in Tables 2 and 3 for the T2J004V3LYEAST strain and the control strain, respectively.

**[Table 2]**

| Expressed Peptide | Type of Insert | Duration of Permeation Experiment | Number of Yeasts that Permeated |
|---|---|---|---|
| BT-004 | 12-well | 2h | 0 |
| BT-004 | 6-well | 4.5h | 1 |
| BT-004 | 6-well | 2h | 7 |
| BT-004 | 6-well | 3h | 19 |
| BT-004 | 12-well | 6h | 23 |
| BT-004 | 6-well | 2h | 24 |
| BT-004 | 6-well | 3h | 25 |
| BT-004 | 6-well | 3h | 40 |
| BT-004 | 6-well | 2h | 41 |
| BT-004 | 6-well | 4.5h | 58 |
| BT-004 | 12-well | 3h | 115 |
| BT-004 | 12-well | 2h | 136 |
| BT-004 | 12-well | 6h | 223 |
| BT-004 | 6-well | 4.5h | 330 |
| BT-004 | 6-well | 2h | 780 |
| BT-004 | 12-well | 6h | about 1000 |

**[Table 3]**

| Expressed Peptide | Type of Insert | Duration of Permeation Experiment | Number of Yeasts that permeated |
|---|---|---|---|
| Control | 12-well | 6h | 0 |
| Control | 12-well | 2h | 0 |
| Control | 6-well | 3h | 0 |
| Control | 6-well | 4.5h | 0 |
| Control | 12-well | 6h | 1 |
| Control | 6-well | 2h | 1 |
| Control | 6-well | 4.5h | 1 |
| Control | 6-well | 2h | 2 |
| Control | 12-well | 6h | 9 |
| Control | 6-well | 2h | 9 |
| Control | 6-well | 2h | 9 |
| Control | 12-well | 2h | 9 |
| Control | 6-well | 2h | 41 |
| Control | 6-well | 3h | 80 |
| Control | 6-well | 4.5h | 104 |
| Control | 6-well | 3h | 167 |
| Control | 12-well | 3h | 187 |

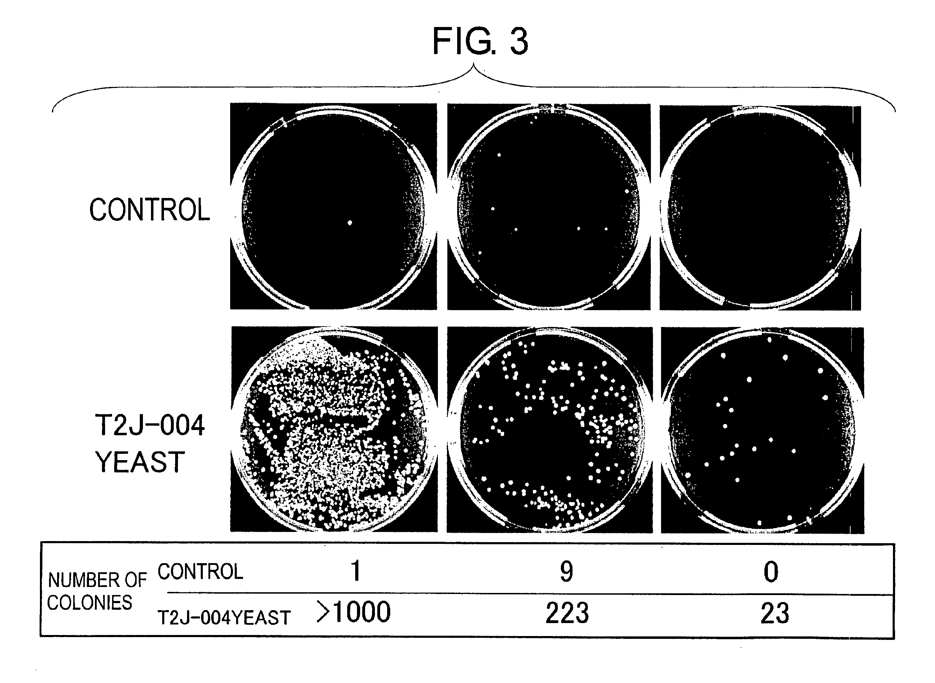

Furthermore, colony formation of each strain is shown in Fig. 3.

As shown above, the results varied in each experiment. However, it was also reported that in a similar permeation experiment in a BBB model with MBEC4 which used, instead of yeasts, microglial cells exhibiting brain-localizing activity, the number of microglial cells that permeated was considerably variable.

The above-mentioned experimental results showed that yeasts expressing (presenting) on the cell surface the polypeptides having brain-localizing activity that were identified by the present inventors, have brain-localizing activity. That is to say, it was discovered that polypeptides can confer brain-localizing activity even to very large molecules such as yeasts.

### Industrial Applicability

The brain-localizing cells of the present invention enable the effective transport of drugs and such into the brain. By using these cells, drugs which have previously been difficult to use, due to metabolic degradation, can be used. Furthermore, the dose of drugs which have previously been used at high concentrations can be reduced.

In addition, the present invention is very useful for the treatment of diseases which require drugs to achieve efficacy in the brain.

The present invention will lead to the development of pharmaceuticals that can bring out maximum drug efficacy, and to the establishment of methods for using them. The present invention may enable the development of novel therapeutic agents for brain diseases, such as brain tumors.

## Claims

1. A brain-localizing cell that expresses a polypeptide having brain-localizing activity on the cell surface.

2. The brain-localizing cell of claim 1, wherein the polypeptide having brain-localizing activity comprises the following amino acid motif sequence:
X₁-(R or K)-X₃-X4; or
X₄-X₃-(R or K)-X₁,
wherein X₁ denotes S, T, N, P, V, or L; X₃ denotes an arbitrary amino acid; and X₄ denotes G, S, T, C, N, L, Q, or Y.

3. The brain-localizing cell of claim 2, wherein the amino acid motif sequence is the following sequence:
X₁-(R or K)-X₃-X₄; or
X₄-X₃-(R or K)-X₁,
wherein X₁ denotes S, T, N, P, or V; X₃ denotes an arbitrary amino acid; and X₄ denotes an uncharged polar amino acid (G, S, T, C, N, Q, or Y).

4. The brain-localizing cell of claim 2, wherein the amino acid motif sequence is the following sequence:
X₁-(R or K)-X₃-X₄; or
X₄-X₃-(R or K)-X₁,
wherein X₁ denotes S, T, P, or L; X₃ denotes an arbitrary amino acid; and X₄ denotes S, T, C, L, or Q.

5. The brain-localizing cell of claim 1, wherein the polypeptide having brain-localizing activity comprises any one of the amino acid sequences of SEQ ID NOs: 1 to 12.

6. The brain-localizing cell of any one of claims 1 to 5, wherein the length of the polypeptide having brain-localizing activity is 9 amino acids or shorter.

7. The brain-localizing cell of any one of claims 1 to 6, wherein the cell has the ability to permeate the blood-brain barrier.

8. The brain-localizing cell of any one of claims 1 to 7, wherein the cell is a yeast cell.

9. A carrier for delivery to the brain which comprises as an active ingredient the brain-localizing cell of any one of claims 1 to 8.

10. A brain-localizing pharmaceutical agent comprising the carrier for delivery to the brain of claim 9 which comprises a biologically active substance.

11. The brain-localizing pharmaceutical agent of claim 10, wherein the biologically active substance has a therapeutic effect on a brain disease.

12. A method for producing a brain-localizing cell, which comprises the step of expressing a polypeptide having brain-localizing activity on the cell surface.

13. The method of claim 12, which comprises the step of introducing a nucleic acid encoding the polypeptide having brain-localizing activity into the cell.

14. A method for producing a brain-localizing pharmaceutical agent, wherein the method comprises the step of introducing a biologically active substance into the brain-localizing cell of any one of claims 1 to 8.

15. A kit for producing a brain-localizing cell, wherein the kit comprises a cell and a nucleic acid encoding a polypeptide having brain-localizing activity as components.

16. A method for translocating a desired substance to the brain of a non-human animal, wherein the method comprises the following steps (a) and (b):
(a) introducing a desired substance into the brain-localizing cell of any one of claims 1 to 8 to produce a brain-localizing cell comprising the substance; and
(b) administering the brain-localizing cell produced in step (a) to the body of a non-human animal.
